# EUROPEAN PATENT SPECIFICATION

(11) **EP 1 552 311 B1**
(45) Date of publication and mention of the grant of the patent: **12.11.2008**
(21) Application number: 03793839.6
(22) Date of filing: 05.09.2003
(51) Int. Cl.: G01N 33/74, G01N 33/573, G01N 33/68

(54) **METHOD FOR DETECTING A RISK OF ACID RELATED DISEASE IN AN INDIVIDUAL**
VERFAHREN ZUM NACHWEIS EINES RISIKOS EINER SÄUREASSOZIIERTEN KRANKHEIT IN EINEM INDIVIDUUM
PROCEDE DE DETECTION D'UN RISQUE DE MALADIE ASSOCIEE A L'ACIDITE CHEZ UN INDIVIDU

(30) Priority: 06.09.2002 FI 20021598
(43) Date of publication of application: 13.07.2005
(73) Proprietor: BIOHIT OYJ, 00880 Helsinki (FI)
(72) Inventor: SUOVANIEMI, Osmo, FIN-00570 Helsinki (FI); HÄRKÖNEN, Matti, FIN-02100 Espoo (FI); SIPPONEN, Pentti, FIN-02160 Espoo (FI)
(74) Representative: Grew, Eva Regina
(86) International application number: PCT/FI2003/000653
(87) International publication number: WO 2004/023148

(56) References cited:
- WO-A1-02/054084
- DATABASE MEDLINE [Online] PARK S. M. ET AL.: 'G- and D-cell populations, serum and tissue concentrations of gastrin and somatostatin in patients with peptic ulcer diseases', XP002974372 Database accession no. NLM7903552 & THE KOREAN JOURNAL OF INTERNAL MEDICINE vol. 8, no. 1, 1993, KOREA, pages 1 - 7
- KEN HARUMA ET AL.: 'Negative Association between Helicobacter Pylori Infection and Reflux Esophagitis in Older Patients: Case-Control Study in Japan' HELICOBACTER vol. 5, no. 1, 2000, pages 24 - 29, XP002974373
- KINOSHITA ET AL.: 'Helicobacter pylori independent chronological change in gastric acid secretion in the Japanese' GUT vol. 41, 1997, pages 452 - 458, XP002974374

## Description

### FIELD OF THE INVENTION

The present invention is directed to a method for detecting a risk of gastric acid related disease in an individual, namely a method for detecting a risk of esophagitis and Barrett's esophagus with associated risk of adenocarcinoma. The method according to the invention is based on assaying the analytes pepsinogen I, a marker for *Helicobacter pylori* and fasting gastrin-17 from a sample, especially a serum sample, of said individual.

### BACKGROUND OF THE INVENTION

Reflux disease is a condition that can be a consequence of the contents of the stomach rising, refluxing, into the esophagus. Specifically the term reflux disease is used when the said reflux phenomenom causes damage or an inflammation in the mucus of the esophagus, e.g. esophagitis. Typical symptoms of reflux disease are heartburns.

A complication of extended reflux disease is a disease called Barrett's esophagus, a disease involving epithelial changes of various degrees in the esophagus. It is believed that reflux destroys the epithelium of the esophagus causing a condition that in turn involves an increased risk of adenocarcinoma of the esophagus. (Cotran, Ramzi S. et al., Robbins Pathologic Basis of Disease, 5th Edition, 1994, W.B. Saunders Company, pp. 761-764)

The stomach of an individual with a healthy, that is a non-atrophic mucosa is acidic, normally at a pH of appr. 1 to 2. The acid excretion in the stomach is however tied to the release of gastrin-17, in that gastrin-17 stimulates the release of acid and an increase in the acidity of the stomach leads to a reduction of the release of gastrin-17 from antral G-cells (gastrin cells) in a feed back mechanism and control. This gastrin-17 is especially the gastrin-17 obtained at a basal or fasting situation, because the gastrin-17 which is measured after stimulation, such as after intake of a protein rich meal, increases, due to the neutralizing effect of the meal on the acidity of the stomach.

The publication abstract Riddel R H (1996), American Journal of Surgical Pathology (1996), Vol. 20, Suppl. 1, PG S31-S50, discloses that inflammation is found as a part of gastroesophageal reflux disease (GERD) in the absence of *Helicobacter pylori*, and suggests a biopsy protocol that maximizes the chances of detecting changes of GERD.

The publication WO 96/15456 discloses a method for screening for the risk of cancer by determining the concentration of the analytes pepsinogen I and gastrin-17 from a serum sample of a subject. According to the said publication, the so determined concentration values are then compared to a selected cut-off value and a reference value for each analyte and the results so obtained can be used to evaluate the presence and location of atrophy in the stomach and associated risk of cancer.

According to an embodiment disclosed, the said tests may be combined with a test for *Helicobacter pylori* antibodies as well as a so-called protein stimulation test, according to which a blood sample is taken in the morning after fasting, whereafter the patient eats a protein-rich standard meal and blood samples are taken at 15 minute intervals for two hours. The maximal increase in gastrin-17 is evident after appr. 20 minutes.

*Helicobacter pylori* is a spiral shaped, gram-negative bacterium which thrives in the mucus in the immediate vicinity of the surface epithelial cells of the gastric mucosa and in the cell interstices. The bacterium apparently is transmitted perorally from one person to the other. The effect of the bacterium on the gastric mucosa is an inflammation reaction, which is mediated over a complement by liberating strong inflammation mediator substances. After the acute stage, the inflammation is transformed into chronic gastritis. In patients suffering from chronic gastritis, in 70 to 90 % a *Helicobacter pylori* infection can be established (Calam, J (1994) Helicobacter pylori (Review) Eur. J. Clin Invest 24: 501-510).

The WO-publication WO 00/67035 discloses a method for assessing the risk of peptic ulcer by determining quantitatively the concentration of serum pepsinogen I and serum gastrin-17 and comparing the obtained values to selected cut-off and reference values. The results so obtained makes it possible to evaluate a possible increased risk of peptic ulcer.

Methods are known in the art for measuring the concentrations of the various analytes, and there are also commercially available kits for this purpose. Some exemplatory methods for carrying out the said determinations are described in the WO-publication 96/15456 as well.

### SUMMARY OF THE INVENTION

According to the invention it has now been discovered that it is possible to detect a risk of esophagitis and Barrett's esophagus in an individual, by determining the analytes pepsinogen I, a marker for *Helicobacter pylori* infection and gastrin-17, which is specifically gastrin-17 obtained in a fasting situation. If it based on the pepsinogen I and Hp-marker determination can be established that the said individual has a non-atrophic and non-gastritic (no *H. pylori* infection) mucosa of the stomach, i.e. the pepsinogen I value is above the cut-off value indicating a healthy corpus mucosa, and the individual does not have a *Helicobacter pylori* infection, for example by establishing a low *Helicobacter pylori* antibody titer, a low fasting gastrin-17 value is indicative of an over-acidic stomach and an increased risk for the individuals to develop acid related disease as defined above.

In the work leading up to the invention it could be established that a low fasting gastrin-17 (G-17fast) level in serum (<1 pmol/l) indicates a highly acidic stomach (acid output is constantly high) which intragastric H⁺ concentration inhibits the release of gastrin-17 from the antral gastrin cells in the stomach. Serum levels of gastrin-17 is an indirect marker of the gastric acidity in subjects in whom the gastric mucosa is normal and healthy, i.e. no *Helicobacter pylori* antibodies and serum pepsinogen I is normal.

It has been established with tests that low fasting gastrin-17 in serum indicates at least a 2fold increase of the risk of acid related diseases, of which can be mentioned e.g., gastroesophageal reflux disease (including those with cardia ulcers and polyps), Barrett's esophagus, duodenal ulcer disease that is not related to *H.pylori* infection, and gastroesophageal reflux disease that develops after eradication of the *H.pylori* infection.

Specifically the present invention is directed to a method for detecting a risk of esophagitis or Barrett's esophagus in an in dividual based on assaying the analytes pepsinogen I, fasting gastrin-17 and a marker for *Helicobacter pylori* infection (Hp-marker) selected from a *Helicobacter pylori* antibody and the *Helicobacter pylori* antigen, the method comprising
- determining the concentration of pepsinogen I and fasting gastrin-17 from a body fluid sample from said individual,
- determining the concentration of a *Helicobacter pylori* antibody from a body fluid sample, or determining the presence of the *Helicobacter pylori* antigen from a stool, biopsy or breath sample, from said individual, and
- comparing the values so determined with a selected cut-off value for respective analyte, whereby
   a pepsinogen I value at or above its respective cut-off value together with a Hp-marker value below its respective cut-off value, in combination with a fasting gastrin-17 value at or below its respective cut-off is indicative of an increased risk of esophagitis or Barrett's esophagus in said individual.

Thus the present invention provides a method which makes it possible to identify an individual with an increased risk to develop an acid related disease as defined above, as well as risk of associated adenocarcinoma.

The method as defined can thus be used as an indirect test for gastric acidity in an individual from a sample, such as a blood, serum or plasma sample.

### DETAILED DESCRIPTION OF THE INVENTION

According to the invention, the process of comparing the measured value to a cut-off value of an analyte is aimed at establishing if the measured value of the analyte is greater than, equal to or smaller than the respective cut-off value.

In the context of the present invention, an individual or a subject means a mammal, such as a human, or an animal, such as a pet, e.g. a dog.

The present invention thus includes in a first step a method for determining at least a *Helicobacter pylori* marker, pepsinogen I (PGI) and fasting gastrin-17 in a sample from the individual to be tested.

The marker or indicator for *Helicobacter pylori* infection can thus, According to the invention, be for example a *Helicobacter pylori* antibody, the value of which can be measured from a body fluid sample. Such a sample is advantageously a serum sample, but can also be a saliva, urine or lacrimal fluid sample, e.g. commercial kits being available for measuring the antibody value. The cut-off value for *Helicobacter pylori* antibodies can easily be determined by a person skilled in the art. In one embodiment of this invention we have used a value of 30 EIU (Enzyme Immunoassay Units) as a cut-off for indicating the presence or absence of a *Helicobacter pylori* infection. Specific antibodies to *Helicobacter pylori* can also be measured using western blot.

Another alternative is to evaluate the presence of a *Helicobacter pylori* infection by determining the presence of the antigen itself. Such measurement can for example be carried out on a stool sample from a patient, and assays, such as enzyme immunoassays are commercially available for this purpose (cf. for example Lancet 1999; 354, 30-33). It is also possible to determine the presence of the antigen from the breath of a patient, using the well known techniques of measuring the carbon dioxide content, the formation of which from labeled urea is catalyzed by *Helicobacter pylori* bacteria, and systems for this assay are also commercially available (e.g. Heliprobe™ by Noster AB, Sweden). Another alternative is to determine the presence or absence of the antigen in a biopsy sample taken during endoscopy from the stomach of a patient. In this alternative the tests are either positive or negative for antigen, and a presence of antigen is taken as being indicative of a *Helicobacter pylori* infection. In the context of this invention, when the Hp-marker is the antigen itself, the test is positive or negative, and the cut-off for infection, being the border between these, can thus be considered to be zero, a positive test being indicative of presence of antigen and consequently the abnormal condition, and a negative test being a value below the cut-off for this marker.

Pepsinogen and gastrin are preferably determined from a body fluid sample, especially from a serum, plasma or whole blood sample, or from a urine, salivary, or lacrimal fluid sample.

The method includes measuring the pepsinogen I analyte, but according to an embodiment of the invention it is also possible the measure, in addition, the concentration of the pepsinogen II analyte (PGII), and using the ratio PGI to PGII instead of PGI, or in addition thereto as the value to be compared to a predetermined cut-off value. Pepsinogen I and also the ratio of pepsinogen I to II tend to decrease linearly with worsening of the atrophy of the corpus mucosa.

In the method according to the invention a cut-off value for pepsinogen I is used, which is the normal cut-off value for differentiating between normal and atrophic corpus mucosa. According to one embodiment of the invention which has given reliable results, a cut-off value for serum or plasma pepsinogen I of ≥ 50 µg/l has been used. This value is somewhat higher than an often used cut-off of 25 µg/l and it can be used in order to ensure that the individual tested certainly has a non-atrophic mucosa. The upper reference limit for serum or plasma pepsinogen I is of the order of 130 µg/l, whereby a value in the vicinity of or above the upper limit can be used as a confirmation of an increased risk of disease. However, a person skilled in the art can easily choose the cut-off value to be used for a specific situation and a specific population to be tested.

Likewise, for gastrin, a cut-off value can be determined, and according to the invention fasting gastrin-17 is measured. According to one embodiment of the invention, a cut-off value of fasting gastrin-17 of ≤ 1 pmol/l has been found suitable for most purposes. Such a value has also proven to be effective in providing a differentiation between individuals at risk and individuals not at risk for developing and acid related disease as defined above.

The concept of cut-off values in assays involving the determination of analyte concentrations is well known to the person skilled in the art. The cut-off value generally means a value or a set of values chosen as a limit between the reference values (normal values) and the abnormal values for the test in question. Such cut-off values are method-specific and depend on the specifity and sensitivity chosen for the test method used for the determination of the analyte concentrations, see for example William J Marshall, Clinical Chemistry, Third Edition, 1995, Mosby.

As mentioned earlier, the methods for measuring pepsinogen I, optionally pepsinogen II and gastrin-17 are well known in the art. The measurements are usually immunological methods, using mono- or polyclonal antibodies to the said analytes.

The detection methods for use include, for example, measuring absorbance, fluoresence or luminescence. It is also possible to carry out all three analyte measurements simultaneously, for example on the same microplate, in different wells or in the same well thereon, which provides for an especially convenient method.

As mentioned earlier there are also commercial kits available for measuring the various analyte concentrations, such as the GastroPanel (Biohit Oyj, Helsinki, Finland) for measuring i.a. pepsinogen I, gastrin-17 and *Helicobacter pylori* antibodies from a blood sample. The measured data can then be assessed for example using software, such as the GastroSoft® software, which draws up a diagnosis and gives recommendation of further treatments based on the results obtained for the analyte concentrations.

### DETAILED DESCRIPTION OF THE DRAWING

In the drawing,
Fig. 1 shows the level of fasting gastrin-17 for Barrett negative and Barrett positive patients with normal stomach. From the results it can be seen that of the Barrett positive patients, 55% had a fasting gastrin-17 value equal to or less than 1 pmol/l, whereas only 23 % of the Barrett negative patients had such a low gastrin-17 value. The difference is statistically significant. Thus the fasting gastrin-17 is a marker distuingishing individuals at risk from individuals not at risk for developing acid related disease in a group of individuals with normal stomach.
Fig. 2 shows the level of fasting gastrin-17 for Barrett negative and Barrett positive patients with normal stomach, and
Fig. 3 the corresponding levels for postprandial gastrin-17 (stimulated gastrin-17). It is evident that fasting gastrin-17 gives a statistically significant differentiation between Barrett negative and Barrett positive patients, whereas the same is not true for postprandial gastrin-17.

### Experimental tests

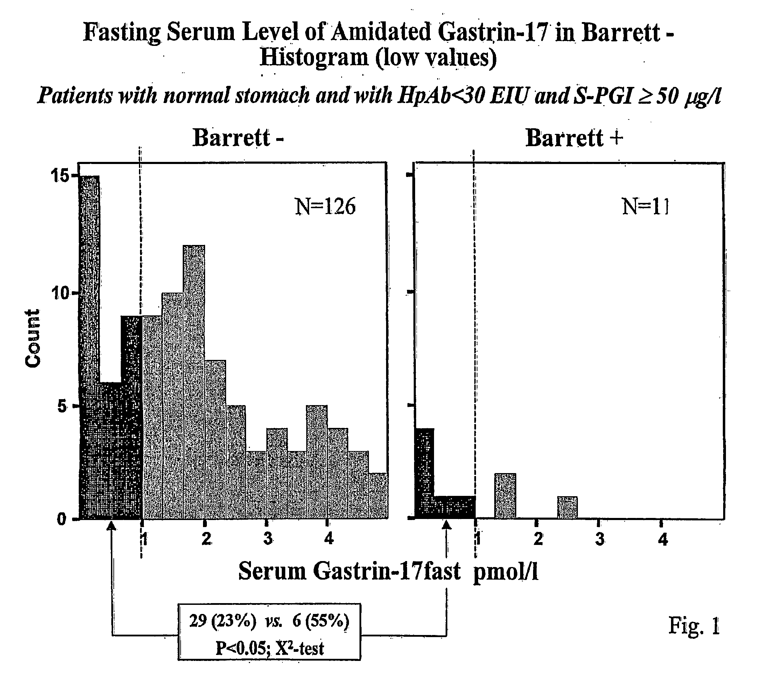

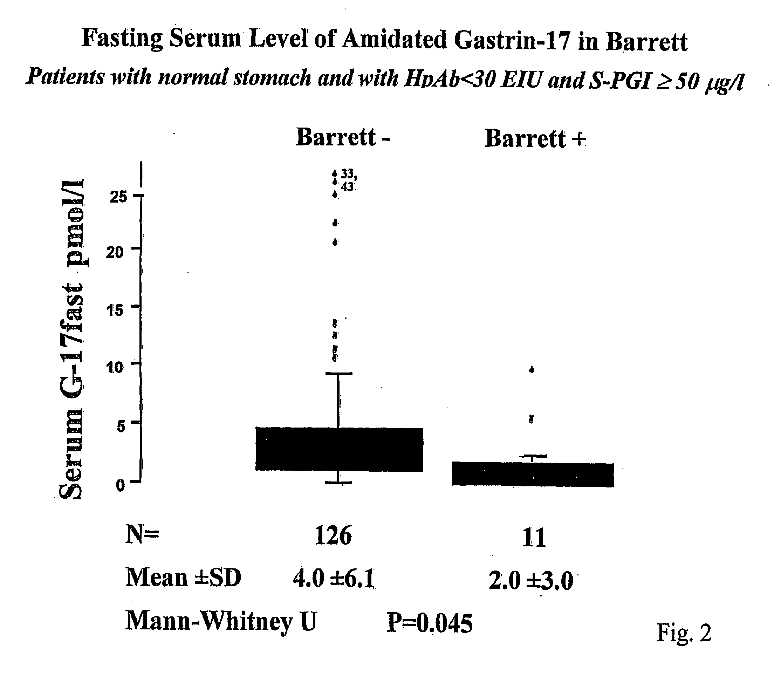

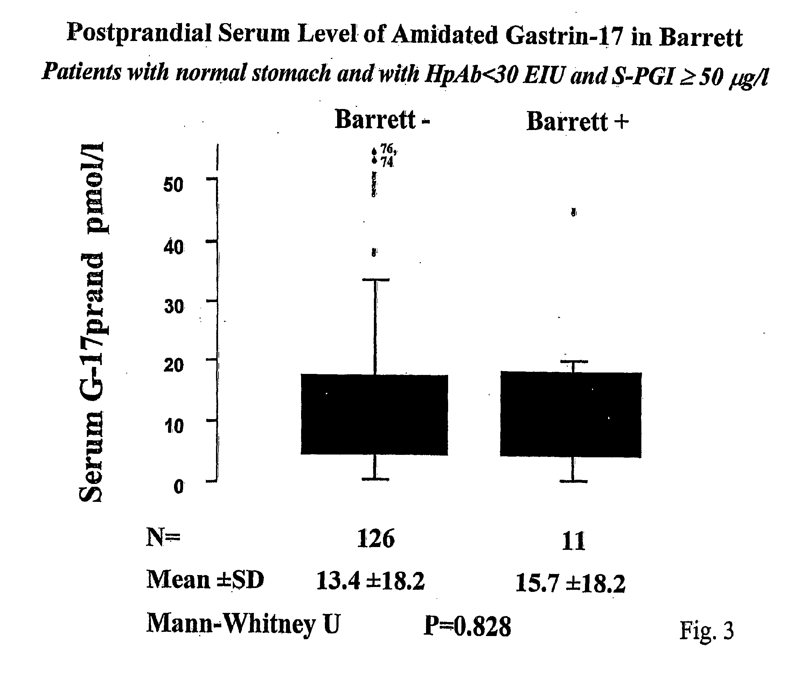

The results shown in Figs 1 to 3 are based on the following tests.

The material tested consisted of 300 endoscopied patients with gastrodyspepsia. They were prospectively collected and two groups of patients were formed. The cases composed of 15 patients with histologically and endoscopically verified classical long segment Barrett. Of these 11 had a normal and healthy gastric mucosa as defined by the absence of *H. pylori* gastritis (*H.pylori* IgG antibody titer <30 EIU) and normal (≥50 µg/l) serum level of pepsinogen I. They also had normal gastric histology in endoscopical biopsies from antrum and corpus. The controls (126 patients) composed of patients without Barrett's esophagus and *H.pylori* antibodies and with normal serum pepsinogen I. These two groups (cases and controls) were compared.

The mean fasting level of amidated gastrin-17 (G-17fast) was significantly (P=0.045 Mann-Whithey U test) lower in patients than in controls (4.0±6.1 pmol/l vs. 2.0±3.0 pmol/l). On the other hand, no differences were between cases and controls in postprandial G-17 levels (G-17prand). Instead, the G-17 prand was slightly higher in Barrett-patients than in controls (15.7±18.2 pmol/l vs 13.4±18.2).

When the 1 pmol/l was set to cut-off limit, 6 of the 11 Barrett patients (55%) had a G 17fast level below this cut-off whereas this prevalence was 29 of 126 (23%) among the controls (P<0.05; chi-square test).

There were, in addition, 5 patients with duodenal ulcer (DU) that occurred in association with a healthy, normal gastric mucosa (the etiology of ulcer in these patients is the usage of NSAIDs or aspirin, in addition to normal or high acid output). The serum level of G 17fast in these 5 DU patients was extremely low (0.458±0.431 pmol/l), i.e., significantly lower than that in appropriate non-DU, non-Barrett controls.

## Claims

1. Method for detecting a risk of esophagitis or Barrett's esophagus in an individual based on assaying the analytes pepsinogen I, fasting gastrin-17 and a marker for *Helicobacter pylori* infection (Hp-marker) selected from a *Helicobacter pylori* antibody and the *Helicobacter pylori* antigen, the method comprising
- determining the concentration of pepsinogen I and fasting gastrin-17 from a body fluid sample from said individual,
- determining the concentration of a *Helicobacter pylori* antibody from a body fluid sample, or determining the presence of the *Helicobacter pylori* antigen from a stool, biopsy or breath sample, from said individual, and
- comparing the values so determined with a selected cut-off value for each respective analyte, whereby a pepsinogen I value at or above its respective cut-off value together with a Hp-marker value below its respective cut-off value, in combination with a fasting gastrin-17 value at or below its respective cut-off is indicative of an increased risk of esophagitis or Barrett' s esophagus in said individual.

2. The method according to claim 1, wherein, in addition, the stimulated gastrin-17 value (G-17st), is measured for comparison.

3. The method according to any one of the preceding claims, wherein, in addition, the concentration of the analyte pepsinogen II (PGII) is measured, and the ratio PGI/PGII is formed for comparison.

4. The method according to claim 1, wherein the body fluid sample is a serum, plasma, whole blood, urine, saliva or lacrimal fluid sample, especially a serum sample.

## Patentansprüche

1. Verfahren zur Feststellung eines Risikos einer Ösophagitis oder eines Barrett-Ösophagus in einer Person, basierend auf der Prüfung der Analyte Pepsinogen I, Nüchtern-Gastrin-17 und eines Markers für *Helicobacter pylori* (Hp-Marker), ausgewählt aus einem *Helicobacter-pylori*-Antikörper und dem *Helicobacter-pylori*-Antigen, wobei das Verfahren folgende Schritte umfasst:
- Bestimmung der Konzentration von Pepsinogen I und Nüchtern-Gastrin-17 aus einer Körperflüssigkeitsprobe der Person,
- Bestimmung der Konzentration eines *Helicobacter-pylori*-Antikörpers aus einer Körperflüssigkeitsprobe oder Bestimmung der Anwesenheit des *Helicobacter-pylori*-Antigens aus einer Stuhl-, Biopsie- oder Atemprobe der Person, und
- Vergleich der auf diese Weise ermittelten Werte mit einem ausgewählten Cut-off-Wert (Grenzwert) für jeden entsprechenden Analyt, wobei
ein Pepsinogen-I-Wert bei oder über seinem entsprechenden Cut-off-Wert in Verbindung mit einem Hp-Marker-Wert unter seinem entsprechenden Cut-off-Wert in Kombination mit einem Nüchtern-Gastrin-17-Wert bei oder unter seinem entsprechenden Cut-off ein erhöhtes Risiko einer Ösophagitis oder eines Barrett-Ösophagus in der Person anzeigt.

2. Verfahren gemäß Anspruch 1, wobei aus Vergleichsgründen zusätzlich der stimulierte Gastrin-17-Wert (G-17st) gemessen wird.

3. Verfahren gemäß einem der vorangehenden Ansprüche, wobei zusätzlich die Konzentration des Analyts Pepsinogen II (PGII) gemessen und aus Vergleichsgründen das Verhältnis PGI/PGII gebildet wird.

4. Verfahren gemäß Anspruch 1, wobei die Körperflüssigkeit eine Serum-, Plasma-, Vollblut-, Urin-, Speichel- oder Tränenflüssigkeitsprobe ist, insbesondere eine Serumprobe.

## Revendications

1. Procédé destiné à détecter un risque d'oesophagite ou de syndrome de Barrett chez un individu sur la base d'un dosage des analytes du pepsinogène I, de la gastrine 17 à jeun et d'un marqueur de l'infection par *Helicobacter Pylori* (marqueur Hp) choisi parmi un anticorps de *Helicobacter Pylori* et l'antigène de *Helicobacter pylori*, le procédé comprenant les étapes consistant à :
- déterminer la concentration du pepsinogène I et de la gastrine 17 à jeun à partir d'un échantillon de liquide biologique provenant dudit individu,
- déterminer la concentration d'un anticorps de *Helicobacter pylori* à partir d'un échantillon de liquide biologique, ou déterminer la présence de l'antigène de *Helicobacter pylori* à partir d'un échantillon de fèces, d'un prélèvement biopsique ou d'un échantillon d'air expiré, provenant dudit individu, et
- comparer les valeurs ainsi déterminées avec une valeur limite choisie pour chaque analyte respectif, moyennant quoi une valeur du pepsinogène I supérieure ou égale à sa valeur limite respective avec une valeur de marqueur Hp inférieure à sa valeur limite respective, en combinaison avec une valeur pour la gastrine 17 à jeun inférieure ou égale à sa valeur limite respective indique un risque accru d'oesophagite ou de syndrome de Barrett chez ledit individu.

2. Procédé selon la revendication 1, dans lequel, en outre, la valeur de la gastrine 17 stimulée (G-17st) est mesurée pour comparaison.

3. Procédé selon l'une quelconque des revendications précédentes, dans lequel, en outre, la concentration du pepsinogène II (PGII) est mesurée, et le rapport PGI/PGII est réalisé pour comparaison.

4. Procédé selon la revendication 1, dans lequel l'échantillon de liquide biologique est un échantillon de sérum, de plasma, de sang total, d'urine, de salive, ou de liquide lacrymal, en particulier un échantillon de sérum.
